# FASCICULE DE BREVET EUROPEEN

(11) **EP 0 606 386 B1**
(45) Date de publication et mention de la délivrance du brevet: **01.05.1996**
(21) Numéro de dépôt: 92921857.6
(22) Date de dépôt: 01.10.1992
(51) Int. Cl.: A61K 7/48, C07C 403/00, C07D 317/20, C07D 317/24, C07D 319/06

(54) **COMPOSITION DERMATOLOGIQUE ET/OU COSMETOLOGIQUE CONTENANT DES RETINOIDES**
RETINOID ENTHALTENDE DERMATOLOGISCHE UND/ODER KOSMETISCHE ZUSAMMENSETZUNGEN
DERMATOLOGICAL AND/OR COSMETOLOGICAL COMPOSITION CONTAINING RETINOIDS

(30) Priorité: 01.10.1991 FR 9112044
(43) Date de publication de la demande: 20.07.1994
(73) Titulaire: Pierre Fabre Dermo-Cosmetique, F-92100 Boulogne (FR)
(72) Inventeur: SAURAT, Jean-Hilaire, CH-1206 Genève (CH); SIEGENTHALER, Georges, CH-1293 Bellevue (CH); COUSSE, Henri, F-31860 Pins-Justaret (FR); MOUZIN, Gilbert, F-31000 Toulouse (FR); GALL, Yvon, F-31120 Portet-sur-Garonne (FR)
(74) Mandataire: Ahner, Francis
(86) Numéro de dépôt international: FR9200908
(87) Numéro de publication internationale: WO9306818

(56) Documents cités:
- EP-A- 0 137 155
- EP-A- 0 391 033

## Description

La présente invention concerne des compositions à usage topique utiles notamment en dermatologie et/ou cosmétologie, ainsi que l'utilisation de rétinoïdes pour la préparation de telles compositions. Elle concerne également un groupe de dérivés rétinoïdes.

En effet, il est communément admis que le rétinol ou (ROL) est essentiel pour la vision, la reproduction, la croissance et la différenciation cellulaire ; par contre, l'acide rétinoïque (ou AR) n'est impliqué que dans la régulation et le contrôle ou la différentiation de la croissance cellulaire (Wolf. G., Physio. Rev. 64, p. 873-937 ; 1984);

L'acide rétinoïque est présent dans le plasma à un taux d'environ 150 fois inférieur à celui du rétinol (Deleenhefr et coll., J. Lipid. Res., 23, p. 1362 à 1367, 1982).

De plus, l'acide rétinoïque est rapidement éliminé dans l'organisme, ce qui suggère que cet acide est formé à partir du rétinol, près du site de son action biologique. L'acide rétinoïque peut être formé par oxydation enzymatique en deux étapes. Lors de ce processus, le rétinol est oxydé en rétinal, qui est ensuite oxydé en acide rétinoïque. Ces deux étapes nécessitent la présence de co-facteur NAD. Des études récentes ont montré la présence d'un système enzymatique capable de transformer le rétinol en acide rétinoïque dans certains tissus de rongeurs, comme les reins et le foie de rats, ou la peau de souris (Connor M.J. et coll., Biochem J., 244, p. 489-492, 1987).

Cependant, ces études n'ont pas élucidé la question de la spécificité des systèmes enzymatiques impliqués, vis-à-vis de leurs substrats: tous ces tissus contiennent des taux importants d'alcool et d'aldéhyde-déhydrogénases, qui sont capables d'oxyder d'une manière non spécifique un grand nombre d'alcools et d'aldéhydes, dont le rétinol et le rétinal.

Ces études n'ont pas montré si les déhydrogénases impliquées dans l'oxydation ROL ----> AR dans le métabolisme naturel des rétinoïdes sont distinctes de celles impliquées dans le métabolisme de l'alcool.

L'origine de l'acide rétinoïque utilisé dans la cellule épidermique est encore actuellement débattue.

L'acide rétinoïque dans le plasma pourrait être une source ; cependant, du fait qu'il est rapidement métabolisé et éliminé, il est probable que l'acide rétinoïque est formé enzymatiquement à partir du rétinol sur son site d'action. Cependant, les études de l'art antérieur n'ont pas pu élucider la nature exacte du système enzymatique impliqué.

Le mécanisme d'action de l'acide rétinoïque et de ses analogues, dans la cellule, met en jeux des récepteurs nucléaires qui appartiennent à la super famille des récepteurs des hormones thyroxostéroïdiennes qui agissent comme facteurs inductibles par l'acide rétinoïque de la transcription génomique (Krust A. et coll., Proc. Natl., Acad. Sci., U.S.A., 86, 5310-5314, 1989). Toutefois, l'analogie entre le mode d'action des hormones thyroxo-stéroïdiennes et celui de l'acide rétinoïque n'est pas total. En effet, l'acide rétinoïque dans les tissus, nécessite une protéine transporteuse intracellulaire ou CRABP (Cellular Retinoic Acid-Binding Protein) qui joue probablement le rôle de vecteur pour transporter son ligand du cytoplasme au noyau.

L'acide rétinoïque est le métabolite actif du rétinol dans certaines fonctions spécifiques (différenciation cellulaire) car l'acide retinoïque est beaucoup plus actif que le rétinol dans le contrôle de la différenciation cellulaire, ce qui suggère que la conversion du rétinol en acide rétinoïque serait responsable, en partie, de l'activité attribuée au rétinol. C'est une des raisons pour lesquelles l'acide rétinoïque ou ses analogues ont été sélectionnés comme agents thérapeutiques.

Par ailleurs, Creek et coll., (J. Invest. Dermatol., 92, p. 283-289, 1989), ont montré que le rétinol apporté dans le milieu extracellulaire, était métabolisé en rétinol-esters (inactifs) par les kératinocytesen culture. Ottonello S. et coll., (J. Biol. Chem., 262, p. 3975-3981, 1987) ont montré que la membrane plasmique des cellules cibles des rétinoïdes possèdait un système enzymatique extrêmement actif. capable d'estérifier le rétinol pour le désactiver et le stocker dans les membranes. De ce fait, on a tout lieu de penser que l'entrée du rétinol dans la cellule est limitée par un mécanisme de contrôle très actif, passant par les estérases. Ces faits expliquent la faible efficacité du rétinol comme agent thérapeutique en dermatologie.

L'acide rétinoïque a ainsi été utilisé sous le nom de "vitamine A acide", par exemple dans la demande EP 230 498, dans des compositions destinées à lutter contre le photovieillissement de la peau.

Les rétinoïdes utilisés en dermatologie sont classiquement l'acide rétinoïque all trans et des analogues tels que l'acide 13 Z rétinoïque, l'acitrétine, l'acide arotinoïque, etc..., et possèdent une réelle activité biologique. Mais ils sont pourvus d'effets secondaires importants, qu'ils soient utilisés par voie systémique ou par voie topique et provoquent notamment une forte irritation locale.

Des composés apparentés ont été utilisés dans différentes compositions. Ainsi, EP 339 905 décrit des compositions cicatrisantes contenant une association d'au moins un facteur de croissance polypeptidique et au moins un retinoïde.

EP 71 537 décrit des compositions pharmaceutiques dans lesquelles sont associes nécessairement un acide linolénique ou un équivalent, avec un produit de type vitamine A, pour le traitement de troubles allergiques et inflammatoires.

EP 391 033 décrit l'utilisation de rétinoïdes pour le traitement d'acné ou de troubles liés au vieillissement.

L'utilisation de nouveaux intermédiaires dans la biosyntnése de l'acide rétinoïque ou de leurs analogues, en court-circuitant, le métabolisme d'activation du rétinol en acide rétinoïque dans la cellule, permet d'obtenir des molécules ayant des affinités différentes vis-a-vis des récepteurs nucléaires de l'acide rétinoïque, et une activité propre qui n'est pas identique à celle de l'acide rétinoïque.

C'est pourquoi la présente invention a pour objet une composition dermatologique et/ou cosmétique, caractérisée en ce qu'elle contient au moins un retinoïde de formule générale (I) 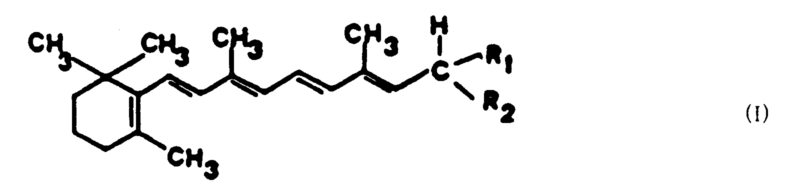
dans laquelle :
R₁ et R₂ représentent indépendamment 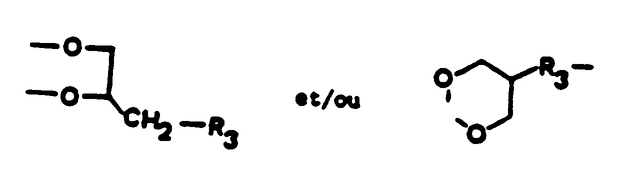
ou R₁ et R₂ représentent ensemble une double liaison avec un atome d'oxygène, ou avec un atome d'azote pour former un groupe imine de formule = N - R₄,
R₃ représente un atome d'hydrogène, un groupe hydroxyle ou un groupe acyloxy à 5 atomes de carbone,
R₄ représente un groupe choisi parmi :

- CH₂ - (CH₂)ₙ - O - R₅ ,

ou 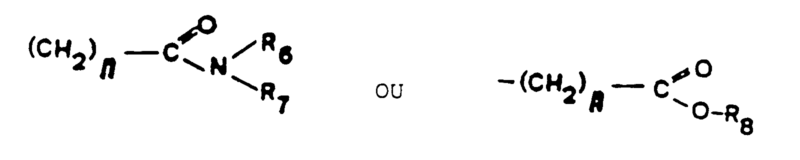
avec n variant de 0 à 5,
R₅ représente un atome d'hydrogène, un groupe alcoyle inférieur de 1 à 5 atomes de carbone, ou un acyl inférieur de 1 à 5 atomes de carbone,
R₆ et R₇ représentent indépendamment un atome d'hydrogène, ou un groupe alcoyle inférieur de 1 à 5 atomes de carbone,
R₈ représente un atome d'hydrogène, ou un groupe alcoyle inférieur de 1 à 5 atomes de carbone.

Les composés répondant à la formule 1 appartiennent au groupe des bioprécurseurs du rétinal.

Le rétinal et/ou ses bioprécurseurs peuvent être oxydés en acide rétinoïque ou réduits en rétinol par des systèmes enzymatiques propres à chaque cellule.

Les bioprécurseurs du rétinal peuvent être oxydés en acide rétinoïque et/ou être transformes en rétinol dans le cas du rétinal et de ses acétals, à l'aide d'enzymes intracellulaires des cellules épidermiques du métabolisme des rétinoïdes. L'utilisation d'une ou plusieurs substances, selon la présente invention, permet d'influencer et de contrôler le métabolisme des rétinoïdes naturels, car la cellule ne possède pas de mécanisme membranaire bloquant leur pénétration. Par ailleurs, elles présentent une toxicité beaucoup moins aigue que l'acide rétinoïque ou ses analogues, ce qui permet leur utilisation également en cosmétologie.

Le métabolisme étant fortement régulé, la mise en contact d'un bioprécurseur du rétinal avec des cellules épidermiques entraîne la biosynthèse d'acide rétinoïque et/ou de rétinol, eux-mêmes pharmacologiquement actifs.

Les compositions, objet de la présente invention, seront utiles pour le traitement de certains troubles dermatologiques et/ou cosmetologiques, sans présenter les effets secondaires des substances utilisées dans l'art antérieur telles que l'acide rétinoïque et ses analogues, ni le manque d'efficacité du rétinol.

En effet, le système enzymatique transforme le rétinol en acide rétinoïque, peut être utilisé comme une étape importante de régulation du métabolisme cellulaire, agissant en amont des récepteurs nucléaires.

Les composés de formule I apparaissent donc comme des produits intermédaires pharmacologiquement actifs. Les tissus, en fonction de leurs diverses activités enzymatiques, pourront transformer les composés de formule I, soit en acide rétinoïque, soit en rétinol, suivant leur besoins spécifiques.

Les rétinoïdes acides agissent directement au niveau génomique par l'intermédiaire des récepteurs nucléaires. De ce fait, la cellule a peu de possibilité pour nuancer l'action des rétinoïdes utilisés jusqu'à présent, mis à part le taux de récepteurs nucléaires, leur catabolisme ou leur biodisponibilité (perméabilité membranaire), ce qui pourrait être responsable des effets secondaires (cheilites, dermatites, etc...).

Les bioprécurseurs préférés sont ceux vis-à-vis desquels la cellule cible ne possède pas de mécanismes membranaires formant barrière à la pénétration. Le rétinal peut à son tour être métabolisé en acide rétinoïque et/ou rétinol. Un avantage de ces bioprécurseurs est de modifier la cinétique globale de la biosynthèse de l'acide rétinoïque et/ou du rétinol, ce qui fournit un moyen de contrôle pharmacologique supplémentaire.

Les bioprécurseurs utilisés selon la présente invention sont les dérivés acétals de formule générale I et plus particulièrement les dérivés de cyclisation avec le glycérol en position 1-2, ou 1-3.

Selon l'un de ses aspects, la présente invention a donc pour objet une composition à usage topique, caractérisée en ce qu'elle renferme l'acétal cyclique 1-2 du glycérol de formule (II) 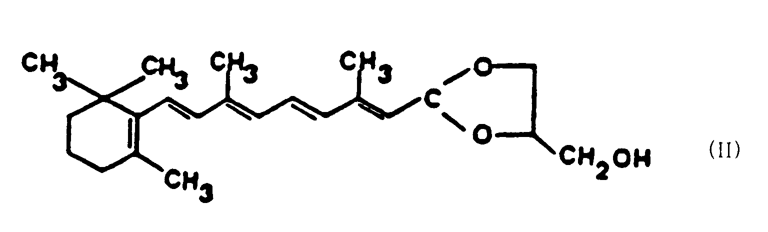
Selon un autre de ses aspects, la présente invention a pour objet une composition à usage topique, caractérisée en ce qu'elle contient le dérivé acétylé de l'acétal cyclique 1-2 du glycérol de formule (III) 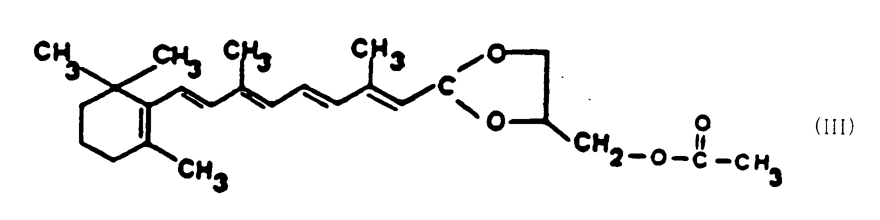
Selon encore un autre de ses aspects, la présente invention à pour objet une composition topique, caractérisée en ce qu'elle contient l'acétal cyclique 1-3 du glycérol de formule (IV)

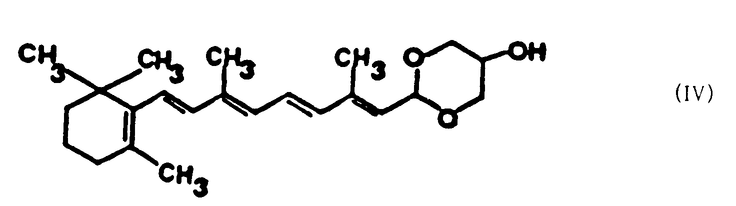

D'autres compositions particulièrement intéressantes selon la présente invention contiennent des imines avec l'aminoéthanol ou la glycinamide, répondant aux formules : 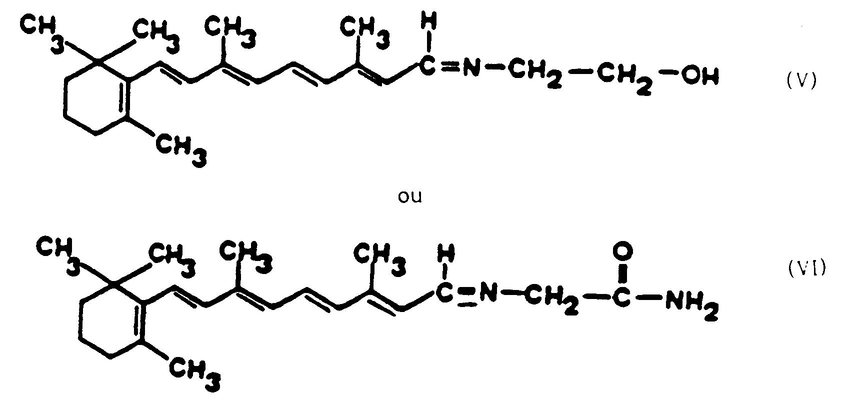
Les compositions selon la présente invention sont notamment utiles en dermatologie, en stomatologie et/ou en cosmétologie.

De manière préférée, les compositions dermatologiques et/ou stomatologiques selon la présente invention, contiennent de 0,01 à 5 % en poids d'au moins un rétinoïde de formule I.

De préférence, le rétinoïde est présent dans la composition a une concentration comprise entre 0,1 et 1 % en poids.

Les rétinoïdes de formule (I) sont, dans un des modes de réalisation de l'invention, utilisés pour la fabrication d'une composition dermatologique, destinée au traitement d'affections choisies dans le groupe comprenant: rosacée, et dermites séborrhéiques.

A cet égard, le rétinal est particulièrement adapté à la préparation de compositions destinées au traitement de la rosacée ou de la dermite séborrhéique.

La rosacée se manifeste notamment par la présence de couperose au niveau du visage. La dermite séborrhéique se caractérise par une atteinte inflammatoire avec desquamation du cuir chevelu, du visage et éventuellement d'autres régions du corps.

L'application de composition contenant du rétinal à des patients présentant ces affections permet une amélioration nette des symptômes observés.

Une autre utilisation des rétinoïdes de formule (I) selon la présente invention, est pour la préparation d'une composition stomatologique, utile dans le traitement des affections des muqueuses, particulièrement des muqueuses buccales.

Selon un autre de ses aspects, la présente invention a pour objet une composition cosmétologique, caractérisée en ce qu'elle contient de 0,01 à 5 % en poids, et de préférence de 0,1 à 1 % d'un rétinoïde de formule I, dans un support cosmétologiquement acceptable.

L'un des objets de la présente invention est donc l'utilisation d'au moins un composé rétinoïde de formule I tel qu'il a été défini ci-dessus, pour la préparation d'une composition cosmétologique.

En effet, un apport exogène de rétinal à une cellule cible va modifier considérablement la redistribution du rétinol et de ses esters, ainsi que le taux d'acide rétinoïque à l'intérieur de la cellule. De plus, cette redistribution se fera suivant les activités enzymatiques propres à la cellule. La Demanderesse a montré que ces activités sont différentes suivant les phases de développement d'une même cellule dans un tissu, ou encore suivant les types de cellules ou de tissus. En outre, la cellule peut encore gérer la dégradation de l'acide rétinoïque ainsi produit afin d'empêcher toute diffusion indésirable à d'autres cellules.

Cette nouvelle technique évite l'arrosage systématique d'un tissu ou d'un organisme par l'acide rétinoïque ou par ses analogues, sans donner la possibilité aux cellules de gérer par leur metabolisme individuel la production des métabolites dont elles ont besoin, par exemple du rétinol ou de l'acide rétinoïque. L'utilisation des rétinoïdes selon la présente invention comme précurseurs pharmacologiquement actifs, permet à la cellule de gérer elle-même ses taux de rétinol ou d'acide rétinoïque selon les besoins du moment.

Les compositions objet de la présente invention auront donc un effet bénéfique, sur toute affection répondant à un traitement par l'acide rétinoïque, en particulier toute affection liée à la différenciation cellulaire, comme par exemple le photovieillissement induit de la peau, ou les précancéroses cutanées, sans présenter les effets secondaires de l'acide rétinoïque.

C'est pourquoi, la présente invention a également pour objet les composés de formule générale I 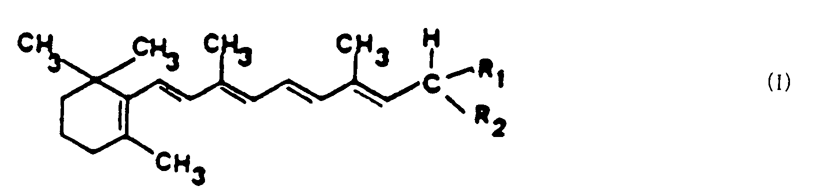
dans laquelle :
R₁ et R₂ représentent indépendamment 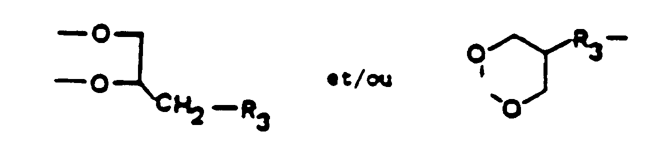
ou R₁ et R₂ représentent ensemble une double liaison avec un atome d'oxygène, ou avec un atome d'azote pour former un groupe imine de formule = N - R₄,
R₃ représente un atome d'hydrogène, un groupe hydroxyle ou un groupe acyloxy à 5 atomes de carbone,
R₄ représente un groupe

- CH₂ - (CH₂)ₙ - O - R₅ ,

ou 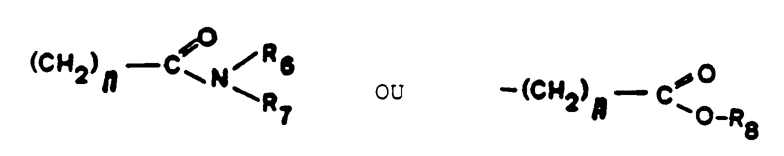
avec n variant de 0 à 5,
R₅ représente un atome d'hydrogène, un groupe alcoyle inférieur de 1 à 5 atomes de carbone, ou un acyl inférieur de 1 à 5 atomes de carbone,
R₆ et R₇ représentent indépendamment un atome d'hydrogène, ou un groupe alcoyle inférieur de 1 à 5 atomes de carbone,
R₈ représente un atome d'hydrogène, ou un groupe alcoyle inférieur de 1 à 5 atomes de carbone.

Les exemples suivants sont destinés à illustrer l'invention sans aucunement en limiter la portée.

### EXEMPLE 1 : ESSAIS IN VITRO

Nous avons mesuré la transformation du rétinol en acide rétinoïque. Nous avons également mesuré l'oxydo-réduction enzymatique du rétinal dans des extraits de cellules épidermiques humaines in vitro (analyse par HPLC des produits obtenus par incubation d'extraits cytosoliques avec RAL et ROL marqués ³H).

Les analyses des rétinoïdes par HPLC sont effectuées par la technique suivante :
Un aliquot de 100 µl de la fraction cytosolique de kératinocytes est incubé avec (³H)-rétinol ou (³H)-rétinal, 600 µM durant une heure.

La réaction est arrêtée par adjonction de 100 µl d'éthanol. Subséquemment, 5 µl de solution standards internes contenant acide 4-oxo-rétinoïque, acide tout-trans-retinoïque, acide 13-cis-rétinoÍque; rétinol et rétinal, sont ajoutés au milieu de réaction.

Le matériel radioactif est ensuite extrait avec 3 x 1 ml d'hexane.

La phase organique est collectée et évaporée complètement par un jet d'azote gazeux.

Le résidu est ensuite repris dans 50 µl d'acétonitrile et analysé sur colonne d'HPLC.

Le système d'HPLC est constitué d'un appareil VARIAN 5000 équipé d'une colonne ODS-Ultrasyl (10 µm, 25 x 0,4 cm) de phase inversée.

Le système d'élution isocratique est constitué d'un mélange de solvants composé de 71 % acétonitrile, 23 % de tampon acétate d'ammonium (50 mM, pH 7) et de 6 % tétrahydrofurane avec un débit de 2,4 ml/min.

Les rétinoïdes non-marqués sont détectés par leur absorbance mesurée à 340 nm. Des fractions de 600 µl sont aussi collectées à l'aide d'un collecteur de fractions et leur radioactivité mesurée avec un compteur à scintillation après addition de 4 ml de Pico-fluor.

En utilisant des concentrations physiologiques de rétinol, on démontre que des extraits cytosoliques de kératinocytes humains, différenciés en culture étaient capables de transformer le rétinol en acide rétinoïque avec une activité enzymatique de 4,49 + 0,17 pmol/h/ mg de protéines, alors que les extraits cytosoliques de kératinocytes humains non differenciés en culture, ne montraient pas d'activité décelable (les kératinocytes sont cultivés par la technique de Rheinwald-Green, Cell.. 6, p. 331-334, 1975 ; la différenciation est contrôlée à l'aide de Ca⁺⁺ exogène).

L'activité de ce système enzymatique dans les cellules épidermiques humaines, diffère de celui qui métabolise l'alcool.

En effet, la Demanderesse a montré que la production d'acide rétinoïque à partir de rétinol n'est pas affectée par des inhibiteurs spécifiques du métabolisme de l'alcool, comme le 4-méthyl-pyrazole et le disulfirame, ni par un excès d'éthanol.

Un autre fait expérimental montrant la spécificité de ce système dans les cellules épidermique est que le cytosol des kératinocytes non-différenciés, en dépit de la présence d'une activité alcool-déshydrogénase de même taux que dans le cytosol des kératinocytes différenciés n'est pas capable de former de l'acide rétinoïque.

Les kératinocytes non-différenciés ne possèdent pas d'activité rétinol déshydrogénase alors qu'ils sont capables de réduire le rétinal en rétinol. La première étape de transformation du rétinol pourrait être régie par deux enzymes différents travaillant chacun dans un sens (réduction ou oxydation) ou bien un seul enzyme capable de travailler réversiblement sont/est responsable de la première étape d'oxydation du rétinol.

Le mécanisme général est schématisé ci-dessous :

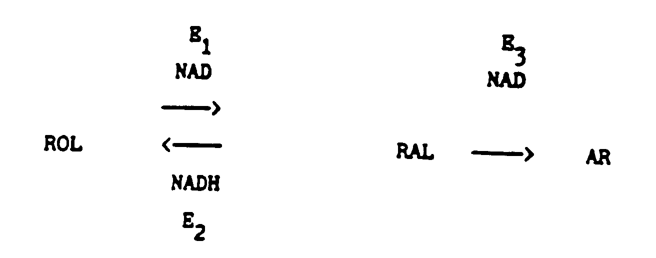

Les activités enzymatiques E₁, E₂, E₃ peuvent montrer de grandes différences entres elles si l'on compare les activités d'une peau normale avec une peau pathologique ou avec des cellules épidermiques (kératinocytes) en culture.

D'après le métabolisme général des rétinoïdes naturels dans la peau, on constate que le rétinal (RAL) joue un rôle central.

En effet, suivant le rapport des cofacteurs NAD/NADH, le RAL peut soit être transformé en AR ou en rétinol, suivant le besoin de la cellule. Par conséquent, le message biologique sera aussi différent. De plus, le RAL semble être un métabolite très acitf car il n'a jusqu'à présent pas été détecté (taux très faible) dans les tissus, alors qu'il apparaît être métabolite intermédiaire dans la production d'AR à partir du ROL.

L'analyse du métabolisme du rétinal montre que dans les cellules épidermiques, le rétinal peut être soit réduit en rétinol ou oxydé en acide rétinoïque. Le sens de la réaction étant influencé par le rapport des cofacteurs NAD/NADH qui entrent en jeu dans les mécanismes enzymatiques d'oxydoréduction. Ainsi, on a montré qu'en présence de NADH, le rétinal est réduit avec la même activité enzymatique dans les extraits de cellules différenciées que dans les extraits de cellules non-différenciées (8 pmol/h/mg) alors que l'oxydation du rétinal est beaucoup plus importante dans les extraits de cellules différenciées (51,6 pmol/h/mg) que dans les extraits de cellules non-différenciées.

La Demanderesse a montré que les extraits de la peau humaine normale et de la plaque de psoriasis sont capables d'oxyder avec la même activité le rétinal en acide rétinoïque, à raison de 1,35 + 0,4 pmol/h/mg. Toutefois, la plaque de psoriasis réduit deux fois plus vite le rétinal en rétinol (2,5 + 0,2 pmol/h/mg) que ne le fait la peau normale (1.0 + 0,3 pmol/h/mg). Ces observations révèlent le rôle central du rétinal comme métabolite intermédiaire soit dans la formation du rétinol, soit dans la formation d'acide rétinoïque et la possibilité d'utiliser RAL pour provoquer la biosynthèse, soit de ROL, soit de AR.

Le rétinal doit être un métabolite très important et déterminant car il se trouve en quantité de traces dans les tissus (il est détecté mais à des concentrations non mesurables dans les tissus embryonnaires (TALLER et coll., Développement. 103, p. 473-483, 1988) et est indétectable dans les tissus adultes (NAPOLI et coll., J. Biol. Chem., 263, p. 17372-17377, 1988 et CONNOR et coll., Biochem. Pharmacol., 36, p. 919-924, 1987) et ceci même chez les animaux traités avec des doses importantes de rétinol.

Le rétinal n'est pas seulement le métabolite intermédiaire de l'oxydation du rétinol en acide rétinoïque, c'est aussi un métabolite du clivage du béta-carotène. Le béta-carotène est une provitamine A dont l'activité vitamine A s'explique par sa transformation en rétinal. NAPOLI et coll. (J. Biol. Chem., 263, p. 17372-17377, 1988) ont montré que des extraits tissulaires (reins, poumons, foie et intestins) de rats transformaient ie béta-carotène plutôt en rétinol qu'en acide-rétinoïque mais ne purent démontrer la présence de rétinal comme métabolite intermédiaire.

La mise en évidence du rétinal dans cette transformation n'a pu être effectuée qu'après la purification partielle de la 15, 15'-béta-carotène dioxygénase. Ces résultats montrent l'importance de l'étape de transformation du rétinal en rétinol, soit pour son stockage sous forme d'esters ou pour toute autre action biologique spécifique, membranaire, second messager, etc...

### EXEMPLE 2 : ESSAIS IN VIVO

### 1. Application du rétinal sur la peau humaine normale

Cet essai réalisé chez 30 volontaires sains était destiné à évaluer l'existence d'un effet biologique et la tolérance après application topique du rétinal.

Trois concentrations 1,0 %, 0,1 % et 0,05 % o nt été utilisées, en applications couvertes quotidiennes pendant au moins 15 jours et jusqu'à 3 mois chez 10 sujets.

La tolérance des préparations à 0,1 % et 0,05 % est excellente; il n'a été noté aucune irritation ni signe de dermite aux rétinoïdes telle qu'on l'observe avec l'acide rétinoïque topique dès les concentrations de 0,01 %. Il existait pourtant un effet biologique, traduit par une meilleure apparence de la peau, analogue à ce qui est observé après utilisation topique d'AR dans les indications dites "anti-vieillissement".

La préparation à 1 % induisait une coloration jaunâtre ; une irritation est survenue dans seulement 10 % des cas, ce qui est nettement différent de ce qui s'observe avec l'AR de telles concentrations.

Ceci indique que le rétinal peut en effet être utilisé à des concentrations thérapeutiques très élevées et qu'à des concentrations plus faibles il exerce un effet de type "cosmétologique" analogue et distinct de celui de l'acide rétinoïque sans induire d'irritations.

### EXEMPLE 2 : APPLICATION DANS LE CAS DE LA ROSACEE

Cet essai a été réalisé chez 10 sujets présentant les signes cliniques de la rosacée. Une composition topique contenant 0,1% de rétinal a été utilisée en application quotidienne pendant 3 mois.

### Résultats

Les résultats ont été très nets : dans 6 cas sur 10, on observe dès le premier mois une amélioration sensible de la rosacée. Après 3 mois de traitement, 70% des sujets traités ne présentent pratiquement plus d'érythrose caractéristique de la rosacée.

### EXEMPLE 3 : APPLICATION DANS LE CAS DE LA DERMITE SEBOR RHEIQUE

Cet essai a été réalisé chez 10 sujets présentant les signes cliniques de la dermite séborrhéique.

Une composition topique contenant 0,05% de rétinal a été utilisée en application quotidienne pendant 1 mois.

### Résultats

Après un mois de traitement, 80% des sujets traités ne présentent pratiquement plus de signes caractéristiques de la dermite séborrhéique.

## Revendications

1. Utilisation d'un rétinoïde de formule générale (I) 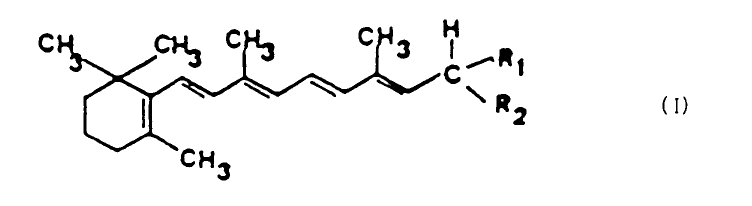 dans laquelle :
R₁ et R₂ représentent indépendamment 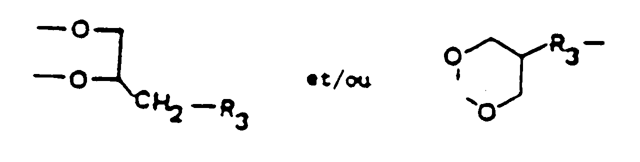 ou R₁ et R₂ représentent ensemble une double liaison avec un atome d'oxygène, ou avec un atome d'azote pour former un groupe imine de formule = N - R₄,
R₃ représente un atome d'hydrogène, un groupe hydroxyle ou un groupe acyloxy à 5 atomes de carbone,
R₄ représente un groupe choisi parmi :
- CH₂ - (CH₂)ₙ - O - R₅ ,
ou 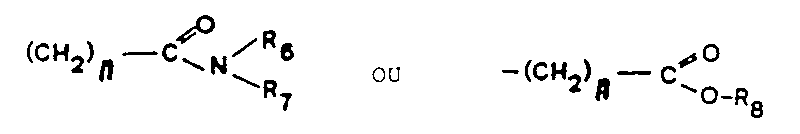 avec n variant de 0 à 5,
R₅ représente un atome d'hydrogène, un groupe alcoyle inférieur de 1 a 5 atomes de carbone, ou un acyl inférieur de 1 à 5 atomes de carbone,
R₆ et R₇ représentent indépendamment un atome d'hydrogène, ou un groupe alcoyle inférieur de 1 à 5 atomes de carbone,
R₈ représente un atome d'hydrogène, ou un groupe alcoyle inférieur de 1 à 5 atomes de carbone,
pour la fabrication d'une composition dermatologique destinée au traitement de la rosacée ou de la dermite séborrhéique.

2. Utilisation d'un rétinoïde de formule générale 1 selon la revendication 1, dans laquelle R₁ et R₂ représentent ensemble une double liaison avec un atome d'oxygène, pour la fabrication d'une composition dermatologique destinée au traitement de la rosacée et/ou de la dermite séborrhéique.

3. Utilisation d'un rétinoïde de formule générale I selon l'une des revendications 1 et 2, caractérisée en ce que la composition contient de 0,01 à 5% du rétinoïde dans un support pharmaceutiquement acceptable.

4. Utilisation d'un rétinoïde selon l'une des revendications 1 et 3, caractérisée en ce que la composition contient de 0,1 à 1% du rétinoïde.

5. Composition dermatologique et/ou cosmétologique, caractérisée en ce qu'elle contient au moins un rétinoïde selon la revendication 1 de formule générale (I) 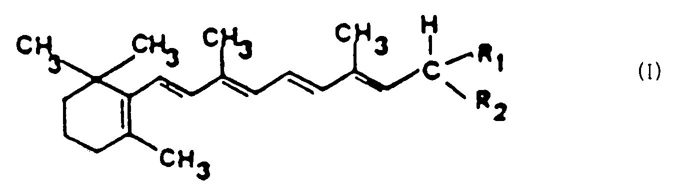 dans laquelle :
R₁ et R₂ représentent indépendamment 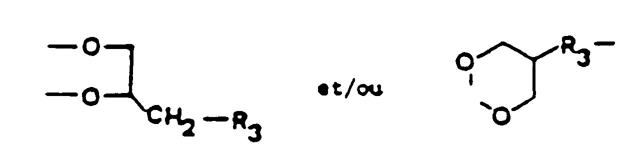 ou R₁ et R₂ représentent ensemble une double liaison avec un atome d'azote pour former un groupe imine de formule = N - R₄,
R₃ représente un atome d'hydrogène, un groupe hydroxyle ou un groupe acyloxy à 5 atomes de carbone,
R₄ représente un groupe choisi parmi :
- CH₂ - (CH₂)ₙ - O - R₅ ,
ou 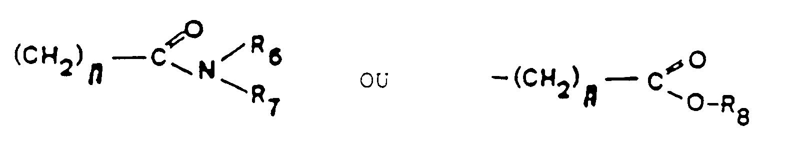 avec n variant de 0 à 5,
R₅ représente un atome d'hydrogène, un groupe alcoyle inférieur de 1 à 5 atomes de carbone, ou un acyl inférieur de 1 à 5 atomes de carbone,
R₆ et R₇ représentent indépendamment un atome d'hydrogène, ou un groupe alcoyle inférieur de 1 à 5 atomes de carbone,
R₈ représente un atome d'hydrogène, ou un groupe alcoyle inférieur de 1 a 5 atomes de carbone.

6. Composition selonla revendication 5, caracterisée en ce qu'elle renferme l'acétal cyclique 1-2 du glycérol de formule (II)

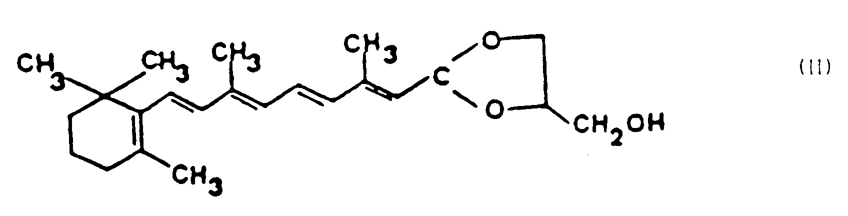

7. Composition selon la revendication 5, caractérisée en ce qu'elle contient le dérivé acétyle de l'acétal cyclique 1, 2 du glycérol de formule (III)

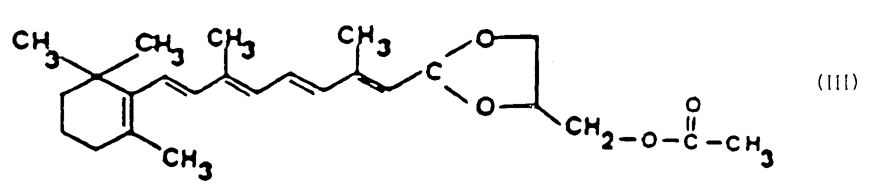

8. Composition selon la revendication 5, caractérisée en ce qu'elle contient l'acétal cyclique 1,3 du glycérol de formule (IV)

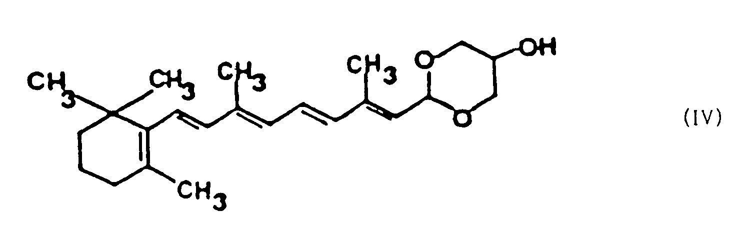

9. Composition selon la revendication 5, caractérisée en ce qu'elle contient un composé formant une fonction imine avec l'aminoéthanol, de formule (V)

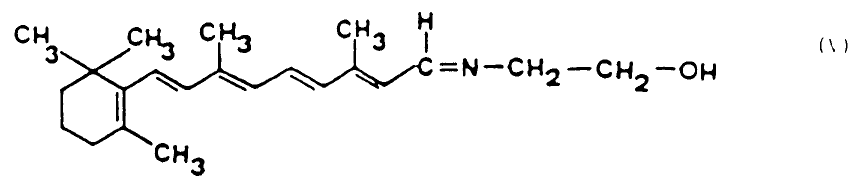

10. Composition selon la revendication 5, caractérisée en ce qu'elle contient le composé formant une fonction imine avec la glycinamide de formule (VI)

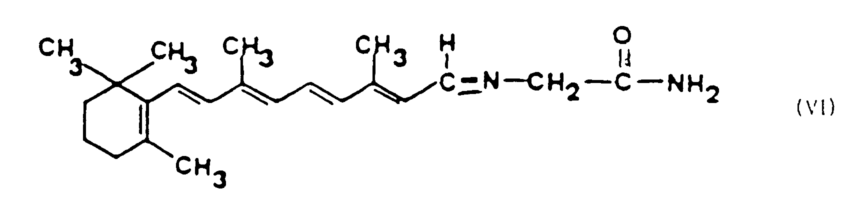

11. Composition dermatologique selon l'une des revendications 5 à 10, caractérisée en ce qu'elle contient 0,01 à 5 % d'au moins un rétinoïde, dans un support pharmaceutiquement acceptable.

12. Composition stomatologique selon l'une des revendications 5 à 10, caractérisée en ce qu'elle contient de 0,01 à 5% d'un rétinoïde selon l'une des revendications 5 à 10, et de préférence de 0,1 à 1% dans un support pharmaceutiquement acceptable, adapté à l'administration sur les muqueuses buccales.

13. Composition cosmétologique selon l'une des revendications 5 à 10, caractérisée en ce qu'elle contient de 0,1 à 1% d'un rétinoïde de formule I, dans un support cosmétologiquement acceptable.

## Claims

1. Use of a retinoid of general formula (I) 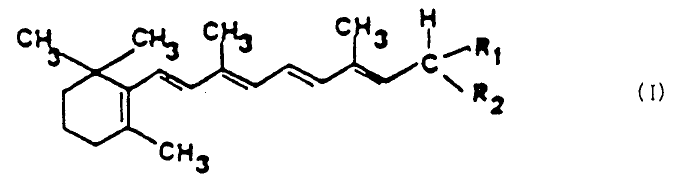 in which:
R₁ and R₂ independently represent 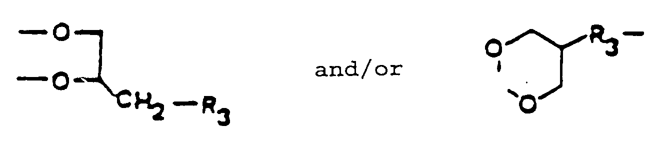 or R₁ and R₂ together represent a double bond with an oxygen atom, or with a nitrogen atom so as to form an imine group of formula = N - R₄,
R₃ represents a hydrogen atom, a hydroxyl group or an acyloxy group containing 5 carbon atoms,
R₄ represents a group chosen from:
-CH₂ - (CH₂)ₙ - O - R₅,
or 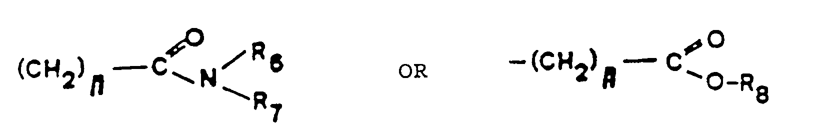 with n ranging from 0 to 5,
R₅ represents a hydrogen atom, a lower alkyl group having 1 to 5 carbon atoms or a lower acyl having 1 to 5 carbon atoms,
R₆ and R₇ independently represent a hydrogen atom or a lower alkyl group having 1 to 5 carbon atoms,
R₈ represents a hydrogen atom or a lower alkyl group having 1 to 5 carbon atoms,
for the manufacture of a dermatological composition intended for the treatment of rosacea or seborrhoeic dermatitis.

2. Use of a retinoid of general formula I according to Claim 1, in which R₁ and R₂ together represent a double bond with an oxygen atom, for the manufacture of a dermatological composition intended for the treatment of rosacea and/or seborrhoeic dermatitis.

3. Use of a retinoid of general formula I according to either of Claims 1 and 2, characterized in that the composition contains from 0.01 to 5 % of the retinoid in a pharmaceutically acceptable vehicle.

4. Use of a retinoid according to either of Claims 1 and 3, characterized in that the composition contains from 0.1 to 1 % of the retinoid.

5. Dermatological and/or cosmetological composition, characterized in that it contains at least one retinoid according to Claim 1, of general formula (I) 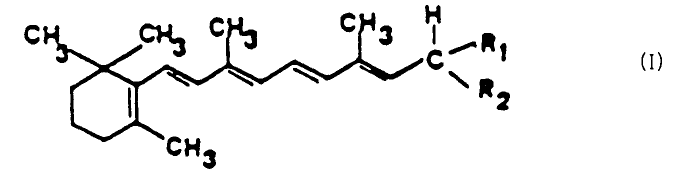 in which:
R₁ and R₂ independently represent 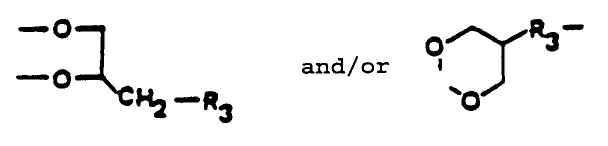 or R₁ and R₂ together represent a double bond with a nitrogen atom so as to form an imine group of formula = N - R₄,
R₃ represents a hydrogen atom, a hydroxyl group or an acyloxy group containing 5 carbon atoms,
R₄ represents a group chosen from:
-CH₂ - (CH₂)ₙ - O - R₅,
or 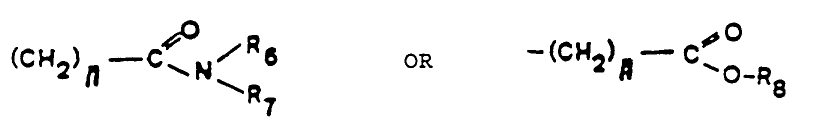 with n ranging from 0 to 5,
R₅ represents a hydrogen atom, a lower alkyl group having 1 to 5 carbon atoms or a lower acyl having 1 to 5 carbon atoms,
R₆ and R₇ independently represent a hydrogen atom or a lower alkyl group having 1 to 5 carbon atoms,
R₈ represents a hydrogen atom or a lower alkyl group having 1 to 5 carbon atoms.

6. Composition according to Claim 5, characterized in that it contains the 1-2 cyclic acetal of glycerol of formula (II)

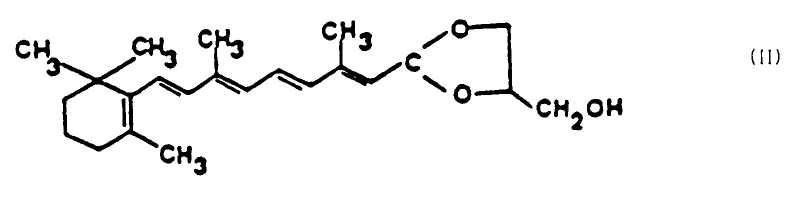

7. Composition according to Claim 5, characterized in that it contains the acetyl derivative of the 1,2 cyclic acetal of glycerol of formula (III)

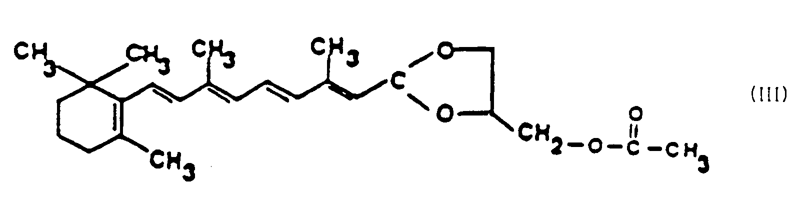

8. Composition according to Claim 5, characterized in that it contains the 1,3 cyclic acetal of glycerol of formula (IV)

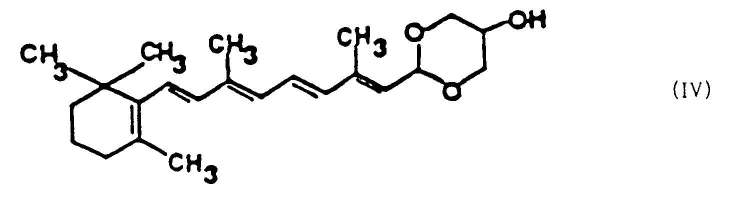

9. Composition according to Claim 5, characterized in that it contains a compound which forms an imine function with the aminoethanol, of formula (V)

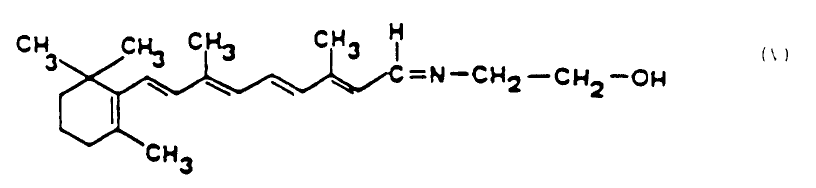

10. Composition according to Claim 5, characterized in that it contains the compound which forms an imine function with the glycinamide of formula (VI)

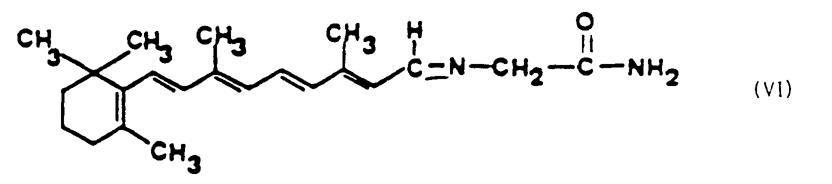

11. Dermatological composition according to one of Claims 5 to 10, characterized in that it contains 0.01 to 5 % of at least one retinoid, in a pharmaceutically acceptable vehicle.

12. Stomatological composition according to one of Claims 5 to 10, characterized in that it contains from 0.01 to 5 % of a retinoid according to one of Claims 5 to 10, and preferably from 0.1 to 1 %, in a pharmaceutically acceptable vehicle, adapted to administration to the buccal mucosae.

13. Cosmetological composition according to one of Claims 5 to 10, characterized in that it contains from 0.1 to 1 % of a retinoid of formula I in a cosmetologically acceptable vehicle.

## Patentansprüche

1. Verwendung eines Retinoids der allgemeinen Formel (I) 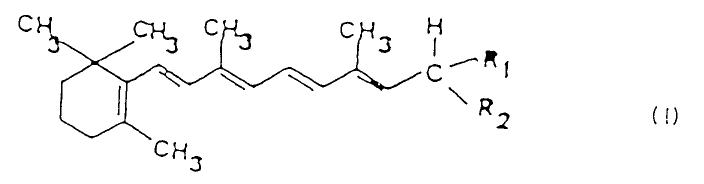 in der bedeuten:
R₁ und R₂ unabhängig voneinander 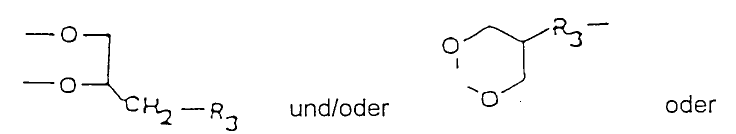 R₁ und R₂ gemeinsam eine Doppelbindung darstellen mit einem Sauerstoffatom oder mit einem Stickstoffatom unter Bildung einer Imin-Gruppe der Formel =N-R₄,
R₃ ein Wasserstoffatom, eine Hydroxylgruppe oder eine Acyloxygruppe mit 5 Kohlenstoffatomen,
R₄ eine Gruppe, ausgewählt aus
- CH₂ - (CH₂)ₙ - O - R₅ ,
oder 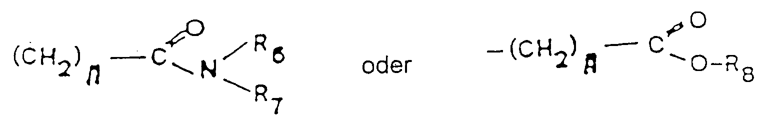 worin n von 0 bis 5 variiert,
R₅ ein Wasserstoffatom, eine niedere Alkylgruppe mit 1 bis 5 Kohlenstoffatomen oder eine niedere Acylgruppe mit 1 bis 5 Kohlenstoffatomen,
R₆ und R₇ unabhängig voneinander ein Wasserstoffatom oder eine niedere Alkylgruppe mit 1 bis 5 Kohlenstoffatomen und
R₈ ein Wasserstoffatom oder eine niedere Alkylgruppe mit 1 bis 5 Kohlenstoffatomen,
zur Herstellung einer dermatologischen Zusammensetzung für die Behandlung der Rosazea oder der seborrhoischen Dermatitis.

2. Verwendung eines Retionids der allgemeinen Formel (I) nach Anspruch 1, in der R₁ und R₂ gemeinsam eine Doppelbindung darstellen mit einem Sauerstoffatom, zur Herstellung einer dermatologischen Zusammensetzung für die Behandlung der Rosazea und/oder der seborrhoischen Dermatitis.

3. Verwendung eines Retionids der allgemeinen Formel (I) nach einem der Ansprüche 1 und 2, dadurch gekennzeichnet, daß die Zusammensetzung 0,01 bis 5 % des Retinoids in einem pharmazeutisch akzeptablen Träger enthält.

4. Verwendung eines Retionids nach einem der Ansprüche 1 und 3, dadurch gekennzeichnet, daß die Zusammensetzung 0,1 bis 1 % des Retinoids enthält.

5. Dermatologische und/oder kosmetische Zusammensetzung, dadurch gekennzeichnet, daß sie mindestens ein Retinoid nach Anspruch 1 der allgemeinen Formel (I) enthält 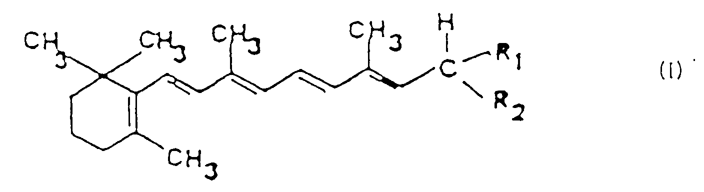 in der bedeuten:
R₁ und R₂ unabhängig voneinander 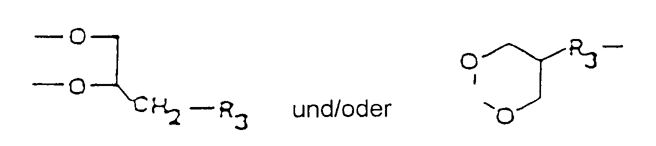 oder worin R₁ und R₂ gemeinsam eine Doppelbindung darstellen mit einem Stickstoffatom unter Bildung einer min-Gruppe der Formel =N-R₄,
R₃ ein Wasserstoffatom, eine Hydroxylgruppe oder eine Acyloxygruppe mit 5 Kohlenstoffatomen,
R₄ eine Gruppe, ausgewählt aus
- CH₂ - (CH₂)ₙ - O - R₅ ,
oder 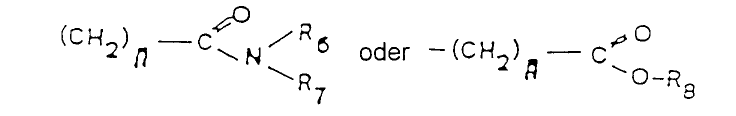 worin n von 0 bis 5 variiert,
R₅ ein Wasserstoffatom, eine niedere Alkylgruppe mit 1 bis 5 Kohlenstoffatomen oder eine niedere Acylgruppe mit 1 bis 5 Kohlenstoffatomen,
R₆ und R₇ unabhängig voneinander ein Wasserstoffatom oder eine niedere Alkylgruppe mit 1 bis 5 Kohlenstoffatomen und
R₈ ein Wasserstoffatom oder eine niedere Alkylgruppe mit 1 bis 5 Kohlenstoffatomen.

6. Zusammensetzung nach Anspruch 5, dadurch gekennzeichnet, daß sie das cyclische Glycerin-1,2-acetal der Formel (II) enthält

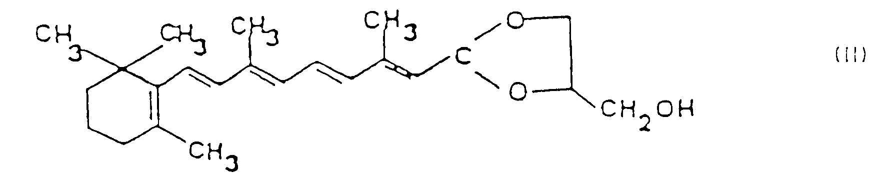

7. Zusammensetzung nach Anspruch 5, dadurch gekennzeichnet, daß sie das Acetylderivat des cyclischen Glycerin-1,2-acetals der Formel (III) enthält

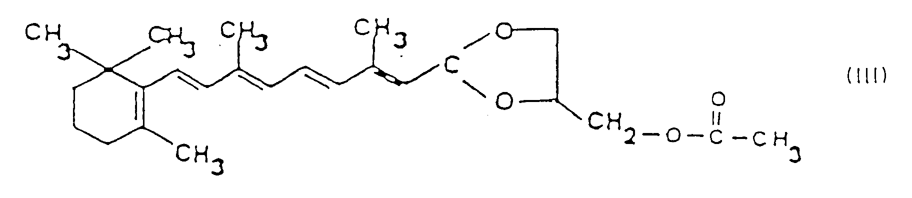

8. Zusammensetzung nach Anspruch 5, dadurch gekennzeichnet, daß sie das cyclische Glycerin-1,3-acetal der Formel (IV) enthält

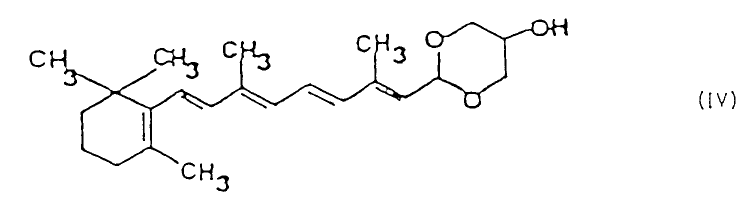

9. Zusammensetzung nach Anspruch 5, dadurch gekennzeichnet, daß sie eine mit Aminoethanol eine Iminfunktion bildende Verbindung der Formel (V) enthält

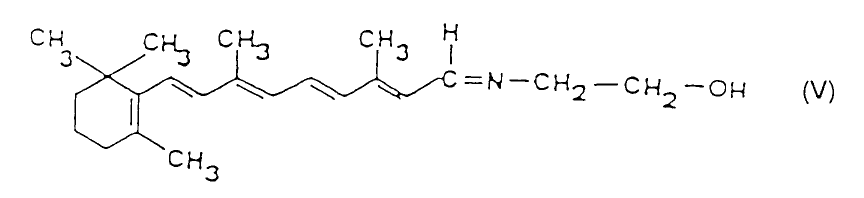

10. Zusammensetzung nach Anspruch 5, dadurch gekennzeichnet daß sie eine mit Glycinamid eine Iminfunktion bildende Verbindung der Formel (VI) enthält

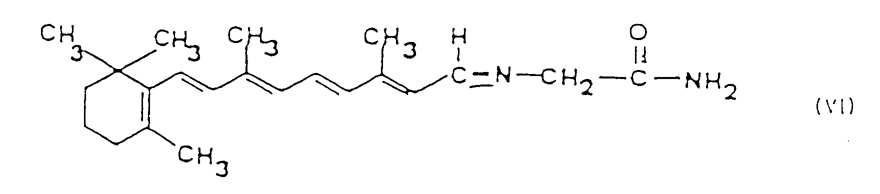

11. Dermatologische Zusammensetzung nach einem der Ansprüche 5 bis 10, dadurch gekennzeichnet, daß sie 0,01 bis 5 % mindestens eines Retinoids in einem pharmazeutisch akzeptablen Träger enthält.

12. Stomatologische Zusammensetzung nach einem der Ansprüche 5 bis 10, dadurch gekennzeichnet, daß sie 0,01 bis 5 %, vorzugsweise 0,1 bis 1 %, eines Retinoids nach einem der Ansprüche 5 bis 10 in einem pharmazeutisch akzeptablen Träger enthält, der für die Verabreichung über die Mundschleimhäute geeignet ist.

13. Kosmetische Zusammensetzung nach einem der Ansprüche 5 bis 10, dadurch gekennzeichnet, daß sie 0,1 bis 1 % eines Retinoids der Formel (I) in einem kosmetologisch akzeptablen Träger enthält.
